# FASCICULE DE BREVET EUROPEEN

(11) **EP 1 064 268 B1**
(45) Date de publication et mention de la délivrance du brevet: **30.11.2005**
(21) Numéro de dépôt: 99907712.6
(22) Date de dépôt: 15.03.1999
(51) Int. Cl.: C07D 233/54, A61K 7/13

(54) **NOUVEAUX COMPOSES CATIONIQUES, LEUR UTILISATION COMME COUPLEURS POUR LA TEINTURE D'OXYDATION DES FIBRES KERATINIQUES, COMPOSITIONS TINCTORIALES ET PROCEDES DE TEINTURE**
KATIONISCHE VERBINDUNGEN, DEREN VERWENDUNG ALS KUPPLER ZUM OXIDATIVEN FÄRBEN KERATINISCHER FASERN, FÄRBEMITTEL UND FÄRBEVERFAHREN
NOVEL CATIONIC COMPOUNDS, THEIR USE AS COUPLING AGENTS FOR OXIDATION DYEING OF KERATINOUS FIBRES, DYEING COMPOSITIONS AND DYEING METHODS

(30) Priorité: 20.03.1998 FR 9803457
(43) Date de publication de la demande: 03.01.2001
(73) Titulaire: L'OREAL, 75008 Paris (FR)
(72) Inventeur: GENET, Alain, F-93600 Aulnay-sous-Bois (FR); LAGRANGE, Alain, F-77770 Coupvray (FR)
(74) Mandataire: Miszputen, Laurent
(86) Numéro de dépôt international: PCT/FR1999/000576
(87) Numéro de publication internationale: WO 1999/048875

(56) Documents cités:
- EP-A- 0 544 400
- WO-A-95/01772
- BE-A- 616 439
- DE-B- 1 135 589
- DE-B- 1 292 784
- FR-A- 1 391 675
- FR-A- 2 520 358
- FR-A- 2 766 178
- TONG L K J ET AL: "The Mechanism of Dye Formation in Color Photography. VII. Intermediate Bases in the Deamination of Quinonediimines" JOURNAL OF THE AMERICAN CHEMICAL SOCIETY., vol. 82, no. 8, 25 avril 1960, pages 1988-1996, XP002060566 DC US

## Description

L'invention a pour objet de nouveaux composés di-benzéniques comportant au moins un groupement cationique Z, Z étant choisi parmi des chaînes aliphatiques quatemisées, des chaînes aliphatiques comportant au moins un cycle saturé quatemisé, et des chaînes aliphatiques comportant au moins un cycle insaturé quatemisé, leur utilisation à titre de coupleur pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet FR-A-2 520 358, d'utiliser certains dérivés cationiques de méta-phénylènediamines, à savoir plus précisément certaines méta-phénylènediamines monosubstituées par une chaîne aliphatique quatemisée, pour la teinture d'oxydation des fibres kératiniques dans des nuances intenses. Toutefois, l'utilisation des méta-phénylènediamines décrites dans cette demande de brevet antérieur ne permet pas d'obtenir une riche palette de couleurs et, de plus, les colorations obtenues ne donnent pas toujours entière satisfaction du point de vue de leur résistance vis à vis des diverses agressions que peuvent subir les cheveux (action de la lumière, de la transpiration, des shampooings, etc...).

Or, la demanderesse vient maintenant de découvrir, de façon totalement inattendue et surprenante, que des composés di-benzéniques de formule (I) ci-après définie, comportant au moins un groupement cationique Z, Z étant choisi parmi des chaînes aliphatiques quatemisées, des chaînes aliphatiques comportant au moins un cycle saturé quatemisé, et des chaînes aliphatiques comportant au moins un cycle insaturé quatemisé, non seulement conviennent pour une utilisation comme coupleur pour la teinture d'oxydation, mais en outre qu'ils permettent d'obtenir des compositions tinctoriales conduisant à des colorations puissantes, dans une très large palette de nuances, et présentant d'excellentes propriétés de résistances aux différents traitements que peuvent subir les fibres kératiniques. Enfin, ces composés s'avèrent être aisément synthétisables.

Ces découvertes sont à la base de la présente invention.

L'invention a donc pour premier objet de nouveaux composés de formule (I) suivante, et leurs sels d'addition avec un acide **tels que définis à la revendication 1 :**

Comme indiqué précédemment, les colorations obtenues avec la composition de teinture d'oxydation conforme à l'invention sont puissantes et permettent d'atteindre des nuances dans une très large palette de couleurs. Elles présentent de plus d'excellentes propriétés de résistance vis à vis de l'action des différents agents extérieurs (lumière, intempéries, lavage, ondulation perrnanente, transpiration, frottements). Ces propriétés sont particulièrement remarquables notamment en ce qui concerne la résistance des colorations obtenues vis à vis de l'action de la lumière, des lavages, de l'ondulation permanente et de la transpiration.

Dans la formule (I) ci-dessus les radicaux alkyle et alcoxy peuvent être linéaires ou ramifiés.

**Dans la formule (IV), deux des radicaux R**_{**10**}**, R**_{**11**} **et R**_{**12**} **peuvent former un cycle pyrrolidinique, un cycle pipéridinique, un cycle pipérazinique ou un cycle morpholinique.**
**Dans la présente invention, X est de préférence choisi parmi un atome d'halogène, un hydroxyde, un hydrogénesulfate, ou un alkyl(C1-C6)sulfate.**

Parmi les composés de formule (I) ci-dessus, on peut notamment citer :
- le dichlorure de 1,4-bis-1-{3-[3-(2,4-diamino-phénoxy)-propyl]-3H-imidazol-1-ium}-butane, monohydrate ;
- le chlorure de 1,3-bis-[3-(2,4-diamino-phénoxy)-propyl]-3H-imidazol-1-ium ;
- le chlorure de 3-[3-(2,4-diamino-phénoxy}-propyl]-1-[(3-hydroxy-4-méthylphénylcarbamoyl)-méthyl]-3H-imidazol-1-ium ;
- le dichlorure de 1,4-bis-{3-[(3-hydroxy-4-méthyl-phénylcarbamoyl)-méthyl]-3H-imidazol-1-ium}-butane ;
- le dichlorure de 1,4-bis-[3-(2,4-diamino-phénoxy)-propyl]-1,4-diméthyl-piperazin-1,4-di-ium ;
- le dichlorure de 1,4-bis-{3-[2-(2,4diamino-phényl)-éthyl]-3H-imidazol-1-ium}-butane ;
- le dichlorure de 1-[3-(2,4-diamino-phénoxy)-propyll-4-[(3-hydroxy-4-méthyl-phénylcarbamoyl)-méthyl]-1,4-diméthyl-pipérazin-1,4-di-ium ;
- le dibromure de 1,4-bis-{3-[2-(3-hydroxy-4-méthyl-phénylamino)-éthyl]-3H-imidazol-1-ium}-butane ;
- le dichlorure de 1,4-bis-(3-[(2,4dihydroxy-phénylcarbamoyl)-méthyl]-3H-imidazol-1-ium}-butane ;
- le chlorure de 3-[3-(2,4-diamino-phénoxy)-propyl]-1-[(2,4dihydroxy-phénylcarbamoyl)-méthyl)-3H-imidazol-1-ium ;
- le bromure, chlorure de 4-[2-(2,4-dihydroxy-phényl)-2-oxo-éthyl]-1-[2-(3-hydroxy-4-méthyl-phénylamino)-éthyl]-1,4-diméthyl-pipérazin-1,4-di-ium ;
- le dibromure de 1,3-bis-{[2-(2,4-diamino-phénoxy)-éthyl]-diéthyl-ammonium}-propane ;
et leurs sels d'addition avec un acide.

Les composés de formule (I) conformes à l'invention peuvent être facilement obtenus, selon des méthodes bien connues de l'état de la technique, par exemple par réduction des composés nitrés cationiques correspondants (méta-nitranilines cationiques ou méta-nitrophénols cationiques).

Cette étape de réduction, (obtention d'une amine aromatique primaire), suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quatemisation) et terminer par la "déprotection" (en général en milieu acide) de la fonction amine.

De même la fonction phénolique peut être protégée selon des procédés bien connus par un radical benzyle ("déprotection" par réduction catalytique) ou par un radical acétyle ou mésyle ("déproteçtion" en milieu acide).

Lorsque la synthèse est terminée, les composés de formule (I) conformes à l'invention peuvent, le cas échéant, être récupérés par des méthodes bien connues de l'état de la technique telles que la cristallisation ou la distillation.

Un autre objet de l'invention est l'utilisation des composés de formule (I) conformes à l'invention à titre de coupleur pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de coupleur, dans un milieu approprié pour la teinture, au moins un composé de formule (I) conforme à l'invention.

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Selon une forme de réalisation préférée de l'invention, la composition tinctoriale renferme en outre une ou plusieurs bases d'oxydation qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le -N,N-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.
Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.
Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlofiydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 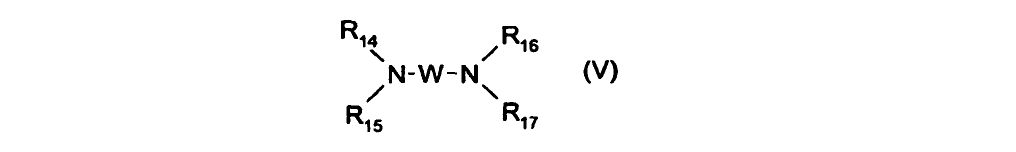 dans laquelle W est un reste propylène éventuellement substitué par un groupement hydroxyle ou un radical alkyle en C₁-C₆ ; R₁₄, R₁₅, R₁₆ et R₁₇, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.
Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases et les oxydo-réductases à 2 électrons. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLE DE PREPARATION

### Synthèse du dichlorure de 1,4-bis-1-{3-[3-(2,4-diamino-phénoxy)-propyl]-3H-imidazol-1-ium}-butane, tétrachlorhydrate, monohydrate

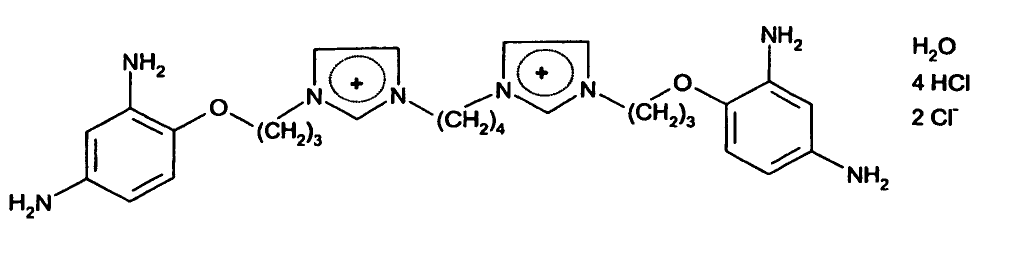

### a) Préparation de synthèse du N-[2-(3-chloro-propoxy)-5-nitro-phényl]-acétamide

Sous agitation on a chauffé à 30-35°C un mélange de 186,5 g (0,94 mole) de N-(2-hydroxy-5-nitro-phényl)-acétamide et de 142,7 g (1,03 mole) de carbonate de potassium dans 570 ml de diméthylformamide; puis on a ajouté 444,0 g (2,82 moles) de 1-bromo-3-chloro-propane et continué de chauffer à 40°C pendant 7 heures (suspension orangée).
On a versé le mélange réactionnel dans 3 litres d'eau glacée, essoré le précipité cristallisé, réempaté dans l'eau puis dans l'alcool isopropylique et séché sous vide à 40°C sur anhydride phosphorique.
Après purification par recristallisation de l'isobutanol au reflux, on a obtenu 203,0 g de cristaux beiges qui ont fondu à 134°C (Kofler) et dont l'analyse élémentaire calculée pour C₁₁H₁₃N₂O₄Cl était :

| % | C | H | N | O | Cl |
|---|---|---|---|---|---|
| Calculé | 48,45 | 4,81 | 10,27 | 23,47 | 13,00 |
| Trouvé | 48,58 | 4,79 | 10,25 | 23,50 | 13,20 |

### b) Quaternisation du N-[2-(3-chloro-oropoxy)-5-nitro-phényl-acétamide

On a chauffé au reflux pendant 15 heures 43,6 g (0,16 mole) de N-[2-(3-chloro-propoxy)-5-nitro-phényl]-acétamide obtenu ci-dessus à l'étape précédente et 15,2 g (0,08 mole) de 1,4-di-imidazol-1-yl-butane dans 130 ml de 2-méthyl-1-propanol.
On a refroidi à température ambiante, décanté le précipité huileux et repris dans de l'éthanol absolu jusqu'à cristallisation.
Après essorage, recristallisation dans l'éthanol à 96° au reflux et séchage à 40°C sur potasse on a obtenu 28,0 g de cristaux jaune pâle qui ont fondu à 135°C (Kofler) et dont l'analyse élémentaire calculée pour C₃₂H₄₀N₈O₈Cl₂ + 2H₂O était :

| % | C | H | N | O | Cl |
|---|---|---|---|---|---|
| Calculé | 49,81 | 5,75 | 14,52 | 20,73 | 9,19 |
| Trouvé | 49,39 | 5,84 | 14,48 | 20,53 | 9,10 |

### c) Préparation du dichlorure de 1,4-bis-1-{3-[3-(2-acétamino-4-amino-phénoxy)-propyl]-3H-imidazol-1-ium}-butane

Dans un hydrogénateur on a placé 27,5 g (0,0356 mole) du composé obtenu ci-dessus à l'étape précédente, 7 g de palladium à 5% sur charbon (contenant 50% d'eau), 150 ml d'éthanol 96° et 150 ml d'eau.
La réduction s'est faite en une 1/2 heure sous une pression d'hydrogène d'environ 8 bars et à une température qui a progressivement été portée à 75°C. Après filtration du catalyseur sous azote, on a évaporé le filtrat à sec sous pression réduite.
Le composé cristallisé obtenu a été purifié par recristallisation d'un mélange éthanol/eau au reflux.
On a obtenu 21,8 g de cristaux blanc cassé qui ont fondu à 106-108°C (Kofler).

### d) Désacétylation du dichlorure de 1,4-bis-1-{3-[3-(2-acétamino-4-amino-phénoxy)propyl]-3H-imidazol-1-ium}-butane

On a chauffé pendant une heure au bain-marie bouillant une solution de 20,2 g (0,0299 mole) de dichlorure de 1,4-bis-1-{3-[3-(2-acétamino-4-amino-phénoxy)-propyt]-3H-imidazol-1-ium}-butane obtenu ci-dessus à l'étape précédente dans 40 ml d'acide chlorhydrique aqueux à 36%.
On a refroidi le mélange réactionnel dans un bain de glace, dilué avec 80 ml d'éthanol absolu, essoré, lavé à l'éthanol absolu et séché à 45°C sous vide et sur potasse.
On a obtenu 18,0 g de cristaux blancs de dichlorure de 1,4-bis-1-{3-[3-(2,4-diamino-phénoxy)-propyl]-3H-imidazol-1-ium}-butane, tétrachlorhydrate, monohydrate qui ont fondu avec décomposition à plus de 260°C (Kofler) et dont l'analyse élémentaire calculée pour C₂₈H₄₄N₈O₂Cl₆ + H₂O était :

| % | C | H | N | O | Cl |
|---|---|---|---|---|---|
| Calculé | 44,52 | 6,14 | 14,83 | 6,35 | 28,16 |
| Trouvé | 44,45 | 6,31 | 14,49 | 6,86 | 28,49 |

On a préparé les compositions tinctoriales suivantes (teneurs en grammes) :

| EXEMPLE | 1 | 2 |
|---|---|---|
| Dichlorure de 1,4-bis-1-{3-[3-(2,4-diamino-phénoxy)propyl]-3H-imidazol-1-ium}-butane, tétrachlorhydrate, monohydrate (Composé de formate (I)) | 1,13 | 1,13 |
| Paraphénylènediamine (Base d'oxydation) | 0,324 | - |
| Para-aminophénol (Base d'oxydation) | - | 0,327 |
| Support de teinture commun | (*) | (*) |
| Eau déminéralisée q.s.p. | 100 g | 100 g |

| | | |
|---|---|---|
| (*) Support de teinture commun : - Alcool éthylique à 96° 18 g - Métabisulfite de sodium en solution aqueuse à 35% 0,68 g - Sel pentasodique de l'acide diéthylènetriaminopentacétique 1,1 g - Ammoniaque à 20% 10,0 g - Eau déminéralisée qs 100 g | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris, naturels ou permanentés, à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| 1 | 10 ± 0,2 | Châtain clair bleu mat |
| 2 | 10 ± 0,2 | Châtain clair rouge cendré |

## Revendications

1. Composés de formule (I) suivante, et leurs sels d'addition avec un acide : 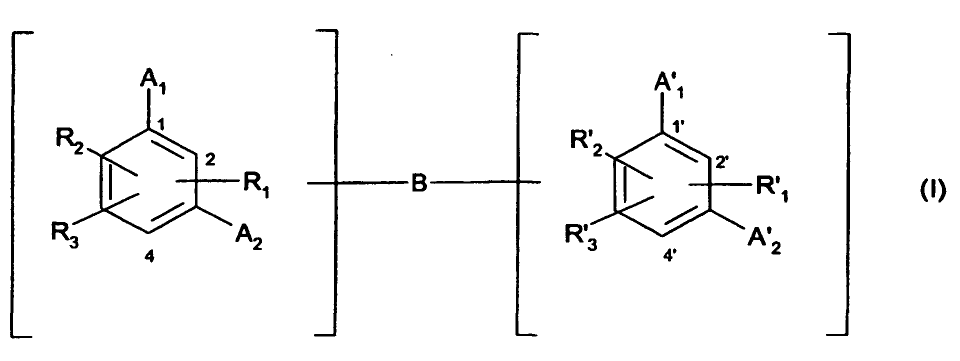 dans laquelle :
• B est un bras de liaison qui représente une chaîne alkylène comportant de préférence de 1 à 14 atomes de carbone, linéaire ou ramifiée pouvant être interrompue par un ou plusieurs hétéroatomes tels que des atomes d'oxygène, de soufre ou d'azote, et éventuellement substituée par un ou plusieurs radicaux hydroxyle ou alcoxy en C₁-C₆ ;
• R₁, R₂, R₃, R'₁, R'₂ et R'₃, qui peuvent être identiques ou différents, représentent un atome d'hydrogène ; un atome d'halogène ; un groupement Z ; un groupe -CO-Z ; un groupe -CO-OZ ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical amino-alkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)rarbamylaikyle(C₁-C₆) ; un radical alkyle en C₁-C₆ ; un radical monohydroxyalkyle en C₁-C₆ ; un radical polyhydroxyalkyte en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; ou un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, lalkyl(C₁-C₆) carboxy, N-alkyl(C₁-C₆)carbamyle, N,N-dialkyl(C₁-C₆)carbamyle, alkyl(C₁-C₆)sulfonyle, aminosulfonyle, N-Z-aminosulfonyle, N-alkyl(C₁-C₆)aminosulfonyle, N,N-dialkyl(C₁-C₆)aminosulfonyle, thiocarbamyle, formyle, un groupe -CO-Z ou par un groupe -CO-OZ ;
• R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, N-alkyl(C₁-C₆)carbamyle, N,N-dialkyl(C₁-C₆)carbamyle, thiocarbamyle, alkyl(C₁-C₆)sulfonyle, et parmi les groupes Z, -CO-Z, ou -CO-OZ ;
• A₁ représente un groupement -NR₄R₅ ou un radical hydroxyle;
• A'₁ représente un groupement -NR'₄R'₅ ou un radical hydroxyle;
• A₂ représente un groupement -NR₇R₈ ou un radical hydroxyle;
• A'₂ représente un groupement -NR'₇R'₈ ou un radical hydroxyle;
• R₄, R₅, R₇, R₈, R'₄, R'₅, R'₇ et R'₈, identiques ou différents, représentent un atome d'hydrogène ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical benzyle; un radical cyanoalkyle en C₁-C₆ ; un radical carbamylalkyle en C₁-C₆; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyte en C₁-C₆ ; un radical sutfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆; un radical aminoalkyle en C₁-C₆ ; un radical aminoalkyle en C₁-C₆ dont l'amine est substituée par un ou deux radicaux identiques ou différents choisis parmi les radicaux alkyle, monohydroxyalkyle en C₁-C₆, polyhydroxyalkyle en C₂-C₆, alkyl(C₁-C₆)carbonyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle;
• un et un seul des radicaux R₄, R₅, R₇, R₈, et/ou un et un seul des radicaux R'₄, R'₅, R'₇ et R'₈ peut également représenter un radical alkyl(C₁-C₆)carboxy ; un radical alkyl(C₁-C₆)carbonyle ; un radical formyle ; un radical trifluoroalkyl(C₁-C₆)carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical N-alkyl(C₁-C₆)carbamyle ; un radical N,N-dialkyl(C₁-C₆)carbamyle ; un radical thiocarbamyle ; un radical aminosuffonyle ; un radical N-alkyl(C₁-C₆)aminosulfonyle ; un radical N,N-dialkyl(C₁-C₆)aminosulfonyle ; un radical alkyl(C₁-C₆)sulfonyle ;
• Z est choisi parmi les groupements cationiques insaturés de formules (II) et (III) suivantes, et les groupements cationiques saturés de formule (IV) suivante : 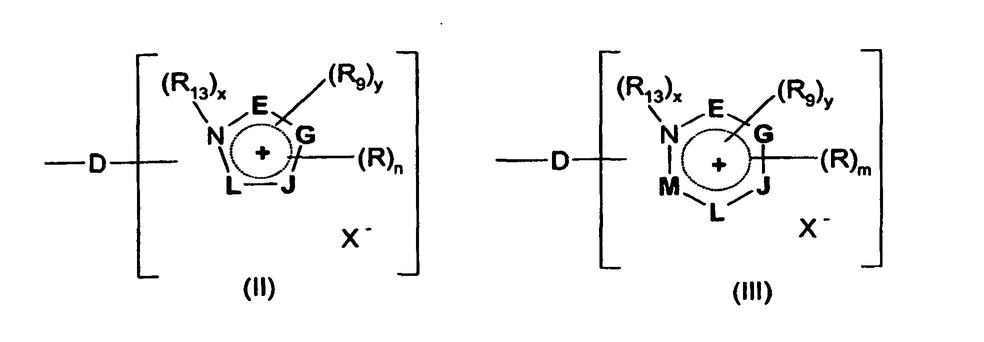 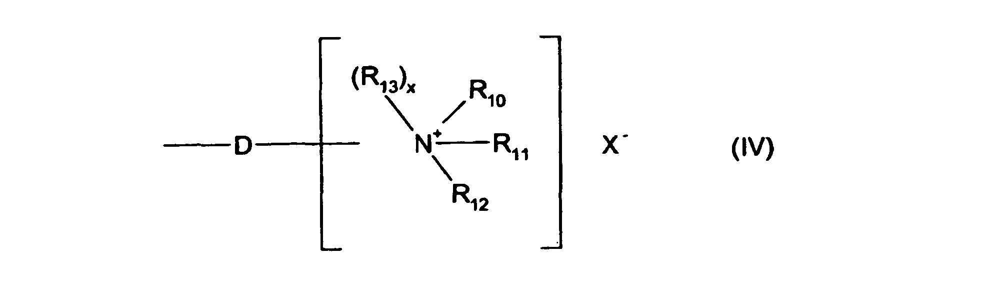 dans lesquelles:
• D est un bras de liaison qui représente une chaîne alkylène comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆ ;
• les sommets E, G, J, L et M, identiques ou différents, représentent les atomes necessaires pour former un cycle pyrrolique, imidazolique, pyrazolique, oxazolique, thlazolique, triazolique, pyrazolopyrimidinium, pyrazolopyridinium, benzoimidazolinium, benzoxazolinium, benzothiazolinium, indolinium, indolidinium, isoindolinium, indazolinium, benzotriazolinium, benzoimidazolidinium, et benzopyrimidinium ; pyridinique, pyrimidinique pyrazinique, oxazinique, triazinique, pyrazolpyrimidinium, quinolinium et tetrahydroquinolinium.
• n est un nombre entier compris entre 0 et 4 inclusivement ;
• m est un nombre entier compris entre 0 et 5 inclusivement ;
• les radicaux R, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ; lorsque n est supérieur ou égal à 2, deux des radicaux R adjacents peuvent également former ensemble un cycle insaturé à 5 ou 6 chaînons, carboné ou contenant un ou plusieurs hétéroatomes ;
• R₉ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)Silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ;
• R₁₀, R₁₁ et R₁₂, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, ou alkyl(C₁-C₆)sulfonyle; deux des radicaux R₁₀, R₁₁ et R₁₂ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)Silanealkyle en C₁-C₆, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, ou alkyl(C₁-C₆)sulfonyle ;
• R₁₃ représente un radical alkyle en C₁-C₆ ; un radical monohydroxyalkyle en C₁-C₆ ; un radical polyhydroxyalkyle en C₂-C₆ ; un radical benzyte ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x et y sont des nombres entiers égaux à 0 ou 1 ; avec les conditions suivantes :
- dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₉ est porté par l'atome d'azote du cycle insaturé ; ou bien
2) lorsqu'au moins un des sommets E, G, J et L représente un atome d'azote sur lequel le radical R₉ est fixé ;
- dans les groupements cationiques insaturés de formule (III) :
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₉ est porté par l'atome d'azote du cycle insaturé ;
- dans les groupements cationiques de formule (IV) :
- lorsque x = 0, alors le bras de liaison est rattaché à l'atome d'azote portant les radicaux R₁₀ à R₁₂,
- lorsque x = 1, alors deux des radicaux R₁₀ à R₁₂ forment conjointement avec l'atome d'azote auquel ils sont rattachés un cycle saturé à 5 ou 6 chaînons tel que défini précédemment, et le bras de liaison B est porté par un atome de carbone dudit cycle saturé ;
• X⁻ représente un anion monovalent ou divalent ;
étant entendu que le nombre de groupement cationique Z est au moins égal à 1.

2. Composés selon l'une quelconque des revendications précédentes, **caractérisés par le fait que** deux des radicaux R₁₀, R₁₁ et R₁₂ forment un cycle pyrrolidinique, un cycle pipéridinique, un cycle pipérazinique ou un cycle morpholinique.

3. Composés selon l'une quelconque des revendications précédentes, **caractérisés par le fait que** X⁻ est choisi parmi un atome d'halogène, un hydroxyde, un hydrogènesulfate, ou un alkyl(C₁-C₆)sulfate.

4. Composés selon l'une quelconque des revendications précédentes, **caractérisés par le fait qu'**ils sont choisis parmi :
- le dichlorure de 1,4-bis-1-{3-[3-(2,4-diamino-phénoxy)-propyl]-3H-imidazol-1-ium}-butane, monohydrate ;
- le chlorure de 1,3-bis-[3-(2,4-diamino-phénoxy)-propyl]-3H-imidazol-1-ium ;
- le chlorure de 3-[3-(2,4-diamino-phénoxy)-propyl]-1-[(3-hydroxy-4-méthyl-phénylcarbamoyl)-méthyl]-3H-imidazol-1-ium ;
- le dichlorure de 1,4-bis-{3-[(3-hydroxy-4-méthyl-phénylcarbamoyl)-méthyl]-3H-imidazol-1-ium}-butane ;
- le dichlorure de 1,4-bis-[3-(2,4-diamino-phénoxy}-propyl]-1,4-diméthyl-piperazin-1,4-di-ium ;
- le dichlorure de 1,4-bis-{3-[2-(2,4diamino-phényl)-éthyl]-3H-imidazol-1-ium}-butane ;
- le dichlorure de 1-[3-(2,4-diamino-phénoxy)-propyl]-4-[(3-hydroxy-4-méthyl-phénylcarbamoylrméthyl]-1,4-diméthyl-pipérazin-1,4-di-ium ;
- le dibromure de 1,4-bis-{3-[2-(3-hydroxy-4-méthyl-phénylamino)-éthyl]-3H-imidazol-1-ium}-butane ;
- le dichlorure de 1,4-bis-{3-[(2,4dihydroxy-phénylcarbamoyl)-méthyl]-3H-imidazol-1-ium}-butane ;
- le chlorure de 3-[3-(2,4-diamino-phénoxy)-propyl]-1-[(2,4dihydroxy-phénylcarbamoyl)-méthyl]-3H-imidazol-1-ium ;
- le bromure, chlorure de 4-[2-(2,4-dihydroxy-phényl)-2-oxo-éthyl]-1-[2-(3-hydroxy-4-méthyl-phénylamino)-éthyl]-1,4-diméthyl-pipérazin-1,4-di-ium ;
- le dibromure de 1,3-bis-{[2-(2,4-diamino-phénoxy)-éthyl]-diéthyl-ammonium}-propane ;
et leurs sels d'addition avec un acide.

5. Utilisation des composés de formule (I) tels que définis à l'une quelconque des revendications précédentes, à titre de coupleur pour la teinture d'oxydation des fibres kératiniques, et en particulier des fibres humaines telles que les cheveux.

6. Composition pour la teinture d'oxydation des fibres kératiniques et en particulier des fibres kératiniques humaines telles que les cheveux, **caractérisée par le fait qu'**elle comprend, dans un milieu approprié pour la teinture, au moins un composé de formule (I) tel que défini à l'une quelconque des revendications 1 à 4, à titre de coupleur.

7. Composition selon la revendication 6, **caractérisée par le fait que** le ou les composés de formule (I) représentent de 0,0005 à 12 % en poids du poids total de la composition tinctoriale.

8. Composition selon la revendication 7, **caractérisée par le fait que** le ou les composés de formule (I) représentent de 0,005 à 6 % en poids du poids total de la composition tinctoriale.

9. Composition selon l'une quelconque des revendications 6 à 8, **caractérisée par le fait qu'**elle renferme un ou plusieurs bases d'oxydation choisies parmi les paraphénylènediamines, les bis-phénylalkylènediamines, les para-aminophénols, les ortho-aminophénols et les bases hétérocycliques.

10. Composition selon la revendication 9, **caractérisée par le fait que** les paraphénylènediamines sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl) amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

11. Composition selon la revendication 9, **caractérisée par le fait que** les bis-phénylalkylènediamines sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

12. Composition selon la revendication 9, **caractérisée par le fait que** les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

13. Composition selon l'une quelconque des revendications 9 à 12, **caractérisée par le fait que** la ou les bases d'oxydation représentent de 0,0005 à 12 % en poids du poids total de la composition tinctoriale.

14. Composition selon la revendication 13, **caractérisée par le fait que** la ou les bases d'oxydation représentent de 0,005 à 6 % en poids du poids total de la composition tinctoriale.

15. Composition selon l'une quelconque des revendications 7 à 14, **caractérisée par le fait qu'**elle renferme un coupleur additionnel choisi parmi les métaphénylènediamines, les méta-aminophénols, les métadiphénols et les coupleurs hétérocycliques, et leurs sels d'addition avec un acide.

16. Composition selon la revendication 15, **caractérisée par le fait que** le ou les coupleurs additionnels représentent de 0,0001 à 10 % en poids du poids total de la composition tinctoriale.

17. Composition selon la revendication 16, **caractérisée par le fait que** le ou les coupleurs additionnels représentent de 0,005 à 5 % en poids du poids total de la composition tinctoriale.

18. Composition selon l'une quelconque des revendications 6 à 17, **caractérisée par le fait que** les sels d'addition avec un acide sont choisis parmi les chlorhydrates, les bromhydrates, les sulfates, les citrates, les succinates, les tartrates, les lactates et les acétates.

19. Composition selon l'une quelconque des revendications 6 à 18, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

20. Composition selon l'une quelconque des revendications 6 à 19, **caractérisée par le fait qu'**elle présente un pH compris entre 3 et 12.

21. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 6 à 20, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

22. Procédé selon la revendication 21, **caractérisé par le fait que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à 2 électrons.

23. Dispositif à plusieurs compartiments, ou "kit" de teinture à plusieurs compartiments, dont un premier compartiment renferme une composition tinctoriale telle que définie à l'une quelconque des revendications 6 à 20 et un second compartiment renferme une composition oxydante.

## Patentansprüche

1. Verbindungen der folgenden Formel (I) und deren Additionssalze mit einer Säure: 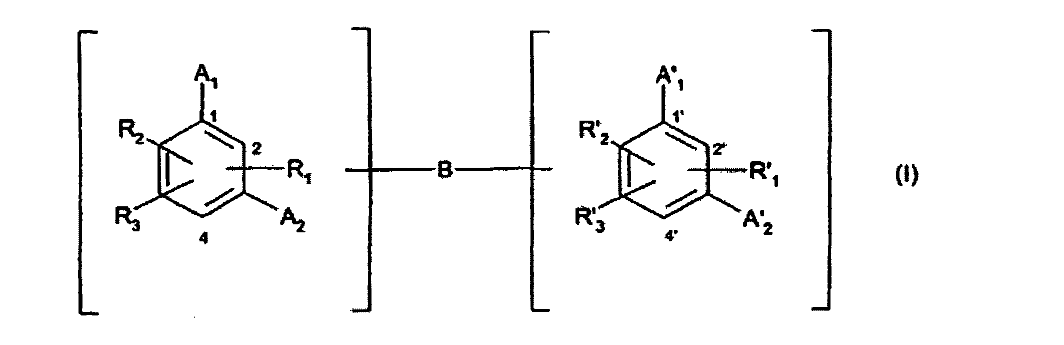 worin bedeuten:
• B eine Verbindungsgruppe, die eine geradkettige oder verzweigte Alkylenkette mit vorzugsweise 1 bis 14 Kohlenstoffatomen bedeutet, die durch ein oder mehrere Heteroatome, wie Sauerstoff, Schwefel oder Stickstoff, unterbrochen und gegebenenfalls mit einer oder mehreren Hydroxygruppen oder C₁₋₆-Alkoxygruppen substituiert sein kann;
• R₁, R₂, R₃, R'₁, R'₂ und R'₃, die gleich oder verschieden sein können, ein Wasserstoffatom; ein Halogenatom; eine Gruppe Z; eine Gruppe -CO-Z; eine Gruppe -CO-OZ; Alkyl(C₁₋₆)-carbonyl; Aminoalkyl(C₁₋₆)carbonyl; N-Z-Aminoalkyl(C₁₋₆)-carbonyl; N-Alkyl(C₁₋₆)aminoalkyl(C₁₋₆)carbonyl; N,N-Dialkyl(C₁₋₆)aminoalkyl(C₁₋₆)carbonyl; Aminoalkyl(C₁₋₆)carbonylalkyl(C₁₋₆); N-Z-Aminoalkyl(C₁₋₆)carbonylalkyl(C₁₋₆); N-Alkyl-(C₁₋₆)aminoalkyl(C₁₋₆)carbonylalk-yl(C₁₋₆); N,N-Dialkyl(C₁₋₆)aminoalkyl(C₁₋₆)carbonylalkyl(C₁₋₆); Carboxy; Alkyl(C₁₋₆)carboxy; Alkyl(C₁₋₆)sulfonyl; Aminosulfonyl; N-Z-Aminosulfonyl; N-Alkyl(C₁₋₆)aminosulfonyl; N,N-Dialkyl(C₁₋₆)aminosulfonyl; Aminosulfonylalkyl(C₁₋₆); N-Z-Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); C₁₋₆-Alkyl; C₁₋₆-Monohydroxyalkyl; C₂₋₆-Polyhydroxyalkyl; Alkoxy(₁₋₆)alkyl(C₁₋₆); C₁₋₆-Trifluoralkyl; Cyano; OR₆ oder SR₆; oder eine Aminogruppe, die mit Alkyl(C₁₋₆)carbonyl, Alkyl(C₁₋₆)carboxy; Trifluoralkyl(C₁₋₆)carbonyl; Aminoalkyl(C₁₋₆)carbonyl, N-Z-Aminoalkyl(C₁₋₆)carbonyl, N-Alkyl(C₁₋₆)aminoalkyl(C₁₋₆)carbonyl; N,N-Dialkyl(C₁₋₆)aminoalkyl(C₁₋₆)carbonyl, Alkyl(C₁₋₆)carboxy; N-Alkyl(C₁₋₆)carbamoyl; N,N-Dialkyl(C₁₋₆)carbamoyl; Alkyl(C₁₋₆)sulfonyl, Aminosulfonyl, N-Z-Aminosulfonyl, N-Alkyl(C₁₋₆)aminosulfonyl; N,N-Dialkyl(C₁₋₆)aminosulfonyl, Thiocarbamoyl, Formyl, -CO-Z oder -CO-OZ geschützt ist;
• R₆ C₁₋₆-Alkyl, C₁₋₆-Monohydroxyalkyl; C₂₋₆-Polyhydroxyalkyl; eine Gruppe Z; Alkoxy(C₁₋₆)alkyl(C₁₋₆); Benzyl; Carboxyalkyl(C₁₋₆); Alkyl(C₁₋₆)carboxyalkyl(C₁₋₆); Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); Trifluoralkyl(C₁₋₆); Aminosulfonylalkyl(C₁₋₆); N-Z-Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carbonylalkyl(C₁₋₆); Aminoalkyl(C₁₋₆); Aminoalkyl(C₁₋₆), wobei die Aminogruppe mit einer oder zwei Gruppen substituiert ist, die gleich oder verschieden und unter den folgenden Gruppen ausgewählt sind: Alkyl(C₁₋₆), Monohydroxyalkyl(C₁₋₆), Polyhydroxyalkyl(C₂₋₆), Alkyl(C₁₋₆)carbonyl, Formyl, Trifluoralkyl(C₁₋₆)carbonyl; Alkyl(C₁₋₆)carboxy, N-Alkyl(C₁₋₆)carbamoyl, N,N-Dialkyl(C₁₋₆)carbamoyl, Thiocarbamoyl, Alkyl(C₁₋₆)sulfonyl und den Gruppen Z, -CO-Z oder -CO-OZ;
• A₁ eine Gruppe -NR₄R₅ oder Hydroxy;
• A'₁ eine Gruppe -NR'₄R'₅ oder Hydroxy;
• A₂ eine Gruppe -NR₇R₈ oder Hydroxy;
• A'₂ eine Gruppe -NR'₇R'₈ oder Hydroxy;
• R₄, R₅, R₇, R₈, R'₄, R'₅, R'₇ und R'8, die gleich oder verschieden sind, ein Wasserstoffatom; C₁₋₆-Alkyl, C₁₋₆-Monohydroxyalkyl; C₂₋₆-Polyhydroxyalkyl; Alkoxy(C₁₋₆)alkyl(C₁₋₆); Benzyl; Cyanoalkyl(C₁₋₆); Carbamoylalkyl(C₁₋₆); N-Alkyl(C₁₋₆) carbamoylalkyl(C₁₋₆); N,N-Dialkyl(C₁₋₆)carbamoylalkyl(C₁₋₆); Thiocarbamoylalkyl(C₁₋₆); Trifluoralkyl(C₁₋₆); Sulfoalkyl(C₁₋₆); Alkyl(C₁₋₆)carboxyalkyl(C₁₋₆); Alkyl(C₁₋₆)sulfinylalkyl(C₁₋₆); Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Alkyl(C₁₋₆)carbonylalkyl(C₁₋₆); Aminoalkyl(C₁₋₆); Aminoalkyl(C₁₋₆), wobei die Aminogruppe mit einer oder zwei Gruppen substituiert ist, die gleich oder verschieden und unter den folgenden Gruppen ausgewählt sind: Alkyl, Monohydroxyalkyl(C₁₋₆), Polyhydroxyalkyl(C₂₋₆), Alkyl(C₁₋₆)carbonyl, N-Alkyl(C₁₋₆)carbamoyl, N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)carboxy oder Thiocarbamoyl;
• wobei eine und nur eine der Gruppen R₄, R₅, R₇, R₈ und/oder eine und nur eine der Gruppen R'₄, R'₅, R'₇ und R'₈ auch Alkyl(C₁₋₆)carboxy; Alkyl(C₁₋₆)carbonyl; Formyl; Trifluoralkyl(C₁₋₆)carbonyl, Aminoalkyl(C₁₋₆)carbonyl; N-Alkyl(C₁₋₆)aminoalkyl(C₁₋₆)carbonyl; N,N-Dialkyl(C₁₋₆)aminoalkyl(C₁₋₆)carbonyl; N-Alkyl(C₁₋₆)carbamoyl; N,N-Dialkyl(C₁₋₆)carbamoyl; Thiocarbamoyl; Aminosulfonyl; N-Alkyl(C₁₋₆)aminosulfonyl; N,N-Dialkyl(C₁₋₆)aminosulfonyl; oder Alkyl(C₁₋₆)sulfonyl bedeuten kann.
• Z ist unter den ungesättigten kationischen Gruppen der folgenden Formeln (II) und (III) und den gesättigten kationischen Gruppen der folgenden Formel (IV) ausgewählt: 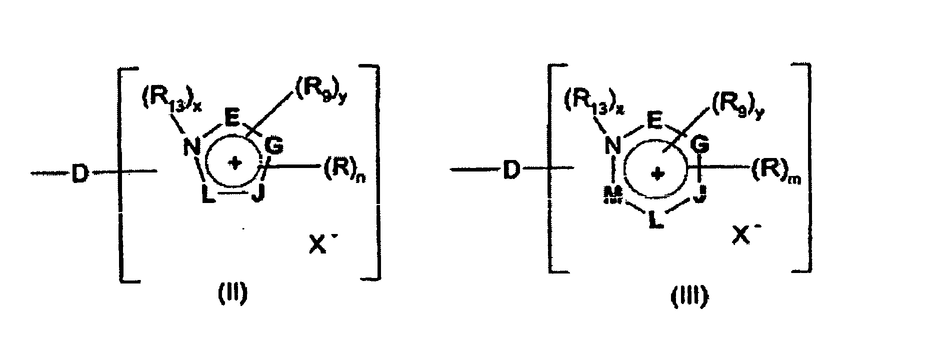 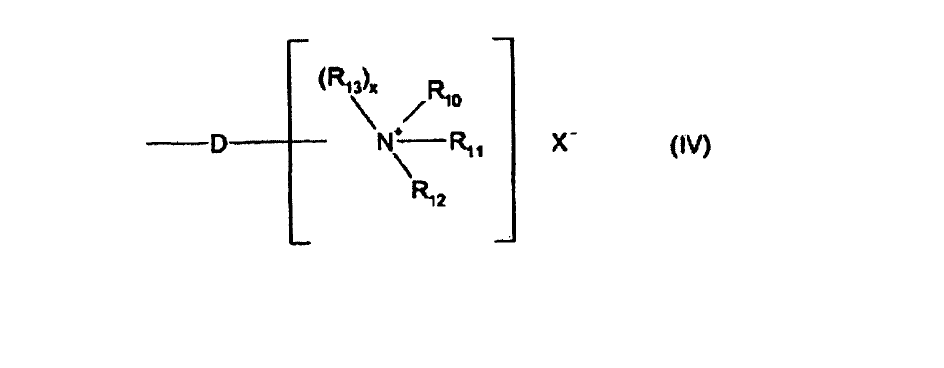
worin bedeuten
• D eine Verbindungsgruppe, die eine geradkettige oder verzweigte Alkylengruppe mit vorzugsweise 1 bis 14 Kohlenstoffatomen sein kann, die durch ein oder mehrere Heteroatome, wie Sauerstoff, Schwefel oder Stickstoff unterbrochen und mit einer und mit mehreren Hydroxygruppen oder C₁₋₆-Alkoxygruppen substituiert sein kann;
• die Ringbestandteile E, G, J, L und M, die gleich oder verschieden sind, solche Atome, die erforderlich sind, um die folgenden Ringe zu bilden: Pyrrol, Imidazol, Pyrazol, Oxazol, Thiazol, Triazol, Pyrazolopyrimidinium, Pyrazolopyridinium, Benzoimidazolinium, Benzoxazolinium, Benzothiazolinium, Indolinium, Indolidinium, Isoindolinium, Indazolinium, Benzotriazolinium, Benzoimidazolidinium und Benzopyrimidinium; Pyridin, Pyrimidin, Pyrazin, Oxazin, Triazin, Pyrazolpyrimidinium, Chinolinium und Tetrahydrochinolinium;
• n 0 oder eine ganze Zahl von 1 bis 4;
• m 0 oder eine ganze Zahl von 1 bis 5;
• die Gruppen R, die gleich oder verschieden sind, ein Halogenatom, Hydroxy, C₁₋₆-Alkyl, C₁₋₆-Monohydroxyalkyl; C₂₋₆-Polyhydroxyalkyl; Nitro, Cyano, C₁₋₆-Cyanoalkyl, C₁₋₆-Alkoxy, Trialkyl(C₁₋₆)silanalkyl(C₁₋₆), Carboxy, C₁₋₆-Alkylcarbonyl, Thio, C₁₋₆-Thioalkyl, Alkyl(C₁₋₆)thio, Amino, eine Aminogruppe, die mit Alkyl(C₁₋₆)carbonyl oder Alkyl(C₁₋₆)sulfonyl geschützt ist, eine Gruppe NHR" oder NR"R"', worin R" und R"', die gleich oder verschieden sind, C₁₋₆-Alkyl, C₁₋₆-Monohydroxyalkyl oder C₂₋₆-Polyhydroxyalkyl bedeuten;
• wenn n 2 bedeutet oder darüber liegt, können zwei angrenzende Gruppen R gemeinsam einen ungesättigten, 5- oder 6-gliedrigen Ring auf Kohlenstoffbasis oder mit einem oder mehreren Heteroatomen bilden;
• R₉ C₁₋₆-Alkyl, C₁₋₆-Monohydroxyalkyl; C₂₋₆-Polyhydroxyalkyl; C₁₋₆-Cyanoalkyl, Trialkyl(C₁₋₆)silanalkyl(C₁₋₆), Alkoxy(C₁₋₆)alkyl(C₁₋₆), Carbamoylalkyl(C₁₋₆), Alkyl(C₁₋₆)carboxyalkyl(C₁₋₆) oder Benzyl;
• R₁₀, R₁₁ und R₁₂, die gleich oder verschieden sind, C₁₋₆-Alkyl, C₁₋₆-Monohydroxyalkyl; C₂₋₆-Polyhydroxyalkyl, Alkoxy(C₁₋₆)alkyl(C₁₋₆), C₁₋₆-Cyanoalkyl, Benzyl, C₁₋₆-Amidoalkyl, Trialkyl(C₁₋₆)silanalkyl(C₁₋₆) oder Aminoalkyl(C₁₋₆), wobei die Aminogruppe mit Alkyl(C₁₋₆)carbonyl oder Alkyl(C₁₋₆)sulfonyl geschützt ist; wobei zwei der Gruppen R₁₀, R₁₁ und R₁₂ auch gemeinsam mit dem Stickstoffatom, an das sie gebunden sind, einen gesättigten, 5- oder 6-gliedrigen Ring auf Kohlenstoffbasis oder mit einem oder mehreren Heteroatomen bilden können, wobei der Ring gegebenenfalls mit einem Halogenatom, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Carboxy, C₁₋₆-Ketoalkyl, Thio, C₁₋₆-Thioalkyl, Alkyl(C₁₋₆)thio, Amino, einer mit Alkyl(C₁-₆)carbonyl oder Alkyl(C₁₋₆)sulfonyl geschützten Aminogruppe substituiert sein kann;
• R₁₃ C₁₋₆-Alkyl; C₁₋₆-Monohydroxyalkyl; C₂₋₆-Polyhydroxyalkyl; Benzyl; C₁₋₆-Aminoalkyl; C₁₋₆-Aminoalkyl, wobei die Aminogruppe mit Alkyl(C₁₋₆)carbonyl, Carbamoyl oder Alkyl(C₁₋₆)sulfonyl geschützt ist; C₁₋₆-Carboxyalkyl; C₁₋₆-Cyanoalkyl; C₁₋₆-Carbamoylalkyl; C₁₋₆-Trifluoralkyl; Trialkyl(C₁₋₆)silanalkyl(C₁₋₆); Sulfonamidoalkyl(C₁₋₆); Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁₋₆)carbamoylalkyl(C₁₋₆); N-Alkyl(C₁₋₆)sulfonamidoalkyl(C₁₋₆);
• x und y 0 oder 1; mit den folgenden Maßgaben:
- in den ungesättigten kationischen Gruppen der Formel (II) :
- die Verbindungsgruppe D ist an das Stickstoffatom gebunden, wenn x = 0,
- die Verbindungsgruppe D ist an ein Atom E, G, J oder L gebunden, wenn x = 1,
- y kann nur den Wert 1 annehmen, wenn:
1) die Atome E, G, J und L gleichzeitig ein Kohlenstoffatom bedeuten und die Gruppe R₉ von dem Stickstoffatom des ungesättigten Rings getragen wird; oder
2) mindestens eines der Atome E, G, J und L ein Stickstoffatom bedeutet, an das R₉ gebunden ist;
- in den ungesättigten kationischen Gruppen der Formel (III) :
- die Verbindungsgruppe D an das Stickstoffatom gebunden ist, wenn x = 0,
- die Verbindungsgruppe D an ein Atom E, G, J oder L oder M gebunden, wenn x = 1,
- y nur den Wert 1 annehmen kann, wenn mindestens ein Atom E, G, L, L und M ein zweiwertiges Atom bedeutet und die Gruppe R₉ von dem Stickstoffatom des ungesättigten Rings getragen wird;
- in den kationischen Gruppen der Formel (IV):
- die Verbindungsgruppe D an das Stickstoffatom gebunden ist, das die Gruppen Rio bis R₁₂ trägt, wenn x = 0,
- zwei der Gruppen Rio bis R₁₂ gemeinsam mit dem Stickstoffatom, an das sie gebunden sind, einen oben definierten gesättigten 5- oder 6-gliedrigen Ring bilden und die Verbindungsgruppe B von einem Kohlenstoffatom des gesättigten Rings getragen wird, wenn x = 1;
• X- ein einwertiges oder zweiwertiges Anion;
mit der Maßgabe, dass die Anzahl der kationischen Gruppen Z mindestens 1 ist.

2. Verbindungen nach einem der vorhergehenden Ansprüche, **dadurch gekennzeichnet, dass** zwei der Gruppen Rio, R₁₁ und R₁₂ einen Pyrrolidinring, Piperidinring, Piperazinring oder Morpholinring bilden.

3. Verbindungen nach einem der vorhergehenden Ansprüche, **dadurch gekennzeichnet, dass** X- unter einem Halogenatom, Hydroxid, Hydrogensulfat oder Alkyl(C₁₋₆)sulfat ausgewählt ist.

4. Verbindungen nach einem der vorhergehenden Ansprüche, **dadurch gekennzeichnet, dass** sie ausgewählt sind unter:
- 1,4-Bis-1-(3-[3-(2,4-diaminophenoxy)-propyl]-3H-imidazol-1-ium}-butandichlorid, Monohydrat;
- 1,3-Bis[3-(2,4-d1anlinophenoxy)-propyl]-3H-imidazol-1 - iumchlorid;
- 3-[3-(2,4-Diaminophenoxy)-propyl]-1-((3-hydroxy-4-methylphenylcarbamoyl)-methyl]-3H-imidazol-1-iumchlorid;
- 1,4-Bis{3-[(3-hydr oxy-4-methylphenylcarbamoyl)-methyl]-3H-imidazol-1-ium}-butandichlorid;
- 1,4-Bis[3-(2,4-diaminophenoxy)-propyl]-1,4-dimethylpiperazin-1,4-diiumdichlorid;
- 1,4-Bis{3-[2-(2,4-diaminophenyl)-ethyl]-3H-imidazol-1-ium}-butandichlorid;
- 1-[3-(2,4-Diaminophenoxy)-propyl]-4-[(3-hydroxy-4-methylphenylcarbamoyl)-methyl]-1,4-dimethylpiperazin-1,4-diiumdichlorid;
- 1,4-Bis{3-[2-(3-hydroxy-4-methylphenylamino)-ethyl]-3H-imidazol-1-ium}-butandibromid;
- 1,4-Bis{3-[2,4-dihydroxyphenylcarbamoyl)-methyl]-3H-imidazol-1-ium}-butandichlorid;
- 3-[3-(2,4-Diaininophenoxy)-propyl]- 1-[(2,4-dihydroxyphenylcarbamoyl)-methyl]-3H-imidazol-1-iumchlorid;
- 4-[2-(2,4-Dihydroxyphenyl)-2-oxo-ethyl]-1-[2-(3-hydroxy-4-methylphenylamino)-ethyl]-1,4-dimethylpiperazin-1,4-diiumchloridbromid;
- 1,3-Bis-{[2-(2,4-diaminophenoxy)-ethyl]-diethylammonium}-propandibromid;
und deren Additionssalzen mit einer Säure.

5. Verwendung von Verbindungen der Formel (I) nach einem der vorhergehenden Ansprüche als Kuppler zum oxidativen Färben von Keratinfasern und insbesondere menschlichen Keratinfasern, wie Haaren.

6. Zusammensetzung zum oxidativen Färben von Keratinfasern und insbesondere menschlichen Keratinfasern, wie Haaren, **dadurch gekennzeichnet, dass** sie in einem zum Färben geeigneten Medium mindestens eine Verbindung der Formel (I) nach einem der Ansprüche 1 bis 4 als Kuppler enthält.

7. Zusammensetzung nach Anspruch 6, **dadurch gekennzeichnet, dass** die Verbindung(en) der Formel (I) 0,0005 bis 12 Gew.-% des Gesamtgewichts der Farbmittelzusammensetzung ausmachen.

8. Zusammensetzung nach Anspruch 7, **dadurch gekennzeichnet, dass** die Verbindung(en) der Formel (I) 0,005 bis 6 Gew.-% des Gesamtgewichts der Farbmittelzusammensetzung ausmachen.

9. Zusammensetzung nach einem der Ansprüche 6 bis 8, **dadurch gekennzeichnet, dass** sie eine oder mehrere Oxidationsbasen enthält, die unter den p-Phenylendiaminen, Bis-phenylalkylendiaminen, p-Aminophenolen, o-Aminophenolen und heterocyclischen Basen ausgewählt sind.

10. Zusammensetzung nach Anspruch 9, **dadurch gekennzeichnet, dass** die p-Phenylendiamine unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methylanilin, N,N-Bis(β-hydroxyethyl)-p-phenylendiamin, 4-N,N-Bis(β-hydroxyethyl)amino-2-methylanilin, 4-N,N-Bis(β-hydroxyethyl)amino-2-chloranilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,y-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin, N-(β-Methoxyethyl)-p-phenylendiamin und deren Additionssalzen mit einer Säure ausgewählt sind.

11. Zusammensetzung nach Anspruch 9, **dadurch gekennzeichnet, dass** die Bisphenylalkylendiaminen unter N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis(4'-amino, 3'-methylphenyl)-ethylendiamin, 1,8-Bis(2,5-diaminophenoxy)-3,5-dioxaoctan und deren Additionssalzen mit einer Säure ausgewählt sind.

12. Zusammensetzung nach Anspruch 9, **dadurch gekennzeichnet, dass** die p-Aminophenole unter p-Aminophenol, 4-Amino-3-methylphenol, 4-Amino-3-fluorphenol, 4-Amino-3-hydroxy-methylphenol, 4-Amino-2-methylphenol, 4-Amino-2-hydroxy-methylphenol, 4-Amino-2-methoxymethylphenol, 4-Amino-2-aminomethylphenol, 4-Amino-2-(β-hydroxyethylaminomethyl)-phenol, 4-Amino-2-fluorphenol und deren Additionssalzen mit einer Säure ausgewählt sind.

13. Zusammensetzung nach einem der Ansprüche 9 bis 12, **dadurch gekennzeichnet, dass** die Oxidationsbase(n) 0,0005 bis 12 Gew.-% des Gesamtgewichts der Farbmittelzusammensetzung ausmachen.

14. Zusammensetzung nach Anspruch 13, **dadurch gekennzeichnet, dass** die Oxidationsbase(n) 0,005 bis 6 Gew.-% des Gesamtgewichts der Farbmittelzusammensetzung ausmachen.

15. Zusammensetzung nach einem der Ansprüche 7 bis 14, **dadurch gekennzeichnet, dass** sie einen zusätzlichen Kuppler enthält, der unter den m-Phenylendiaminen, m-Aminophenolen, m-Dihydroxybenzolen und den heterocyclischen Kupplern und deren Additionssalzen mit einer Säure ausgewählt ist.

16. Zusammensetzung nach Anspruch 15, **dadurch gekennzeichnet, dass** der oder die ergänzende(n) Kuppler 0,0001 bis 10 Gew.-% des Gesamtgewichts der Farbmittelzusammensetzung ausmachen.

17. Zusammensetzung nach Anspruch 16, **dadurch gekennzeichnet, dass** der oder die ergänzende(n) Kuppler 0,005 bis 5 Gew.-% des Gesamtgewichts der Farbmittelzusammensetzung ausmachen.

18. Zusammensetzung nach einem der Ansprüche 6 bis 17, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

19. Zusammensetzung nach einem der Ansprüche 6 bis 18, **dadurch gekennzeichnet, dass** das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethem, aromatischen Alkoholen, analogen Produkten und deren Gemischen ausgewählt ist.

20. Zusammensetzung nach einem der Ansprüche 6 bis 19, **dadurch gekennzeichnet, dass** sie einen pH-Wert im Bereich von 3 bis 12 aufweist.

21. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 6 bis 20 aufgetragen wird und die Farbe bei einem sauren, neutralen oder alkalischen pH-Wert mit einem Oxidationsmittel entwickelt wird, das unmittelbar bei der Anwendung zu der Farbmittelzusammensetzung gegeben wird oder das in einer oxidierenden Zusammensetzung vorliegt, die gleichzeitig oder getrennt davon anschließend aufgetragen wird.

22. Verfahren nach Anspruch 21, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, Enzymen, wie Peroxidasen und Oxidoreduktasen (zwei Elektronen), ausgewählt ist.

23. Vorrichtung mit mehreren Abteilungen oder "Kit" zum Färben mit mehreren Abteilungen, wobei eine erste Abteilung eine Farbmittelzusammensetzung nach einem der Ansprüche 6 bis 20 und eine zweite Abteilung eine oxidierende Zusammensetzung enthält.

## Claims

1. Compounds of formula (I) below, and the addition salts thereof with an acid: 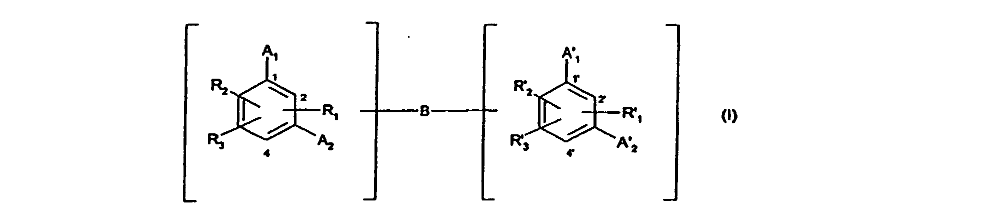 in which:
• B is a linker arm which represents a linear or branched alkylene chain preferably containing from 1 to 14 carbon atoms, which may be interrupted by one or more hetero atoms such as oxygen, sulphur or nitrogen atoms, and optionally substituted with one or more hydroxyl or C₁-C₆ alkoxy radicals;
• R₁, R₂, R₃, R'₁, R'₂ and R'₃, which may be identical or different, represent a hydrogen atom; a halogen atom; a group Z; a group -CO-Z; a group -CO-OZ; a (C₁-C₆)alkylcarbonyl radical; an amino(C₁-C₆)alkylcarbonyl radical; an N-Z-amino(C₁-C₆)alkylcarbonyl radical; an N- (C₁-C₆) alkylamino (C₁-C₆) alkylcarbonyl radical; an N, N-di (C₁-C₆) alkylamino (C₁-C₆) alkylcarbonyl radical; an amino (C₁-C₆) alkylcarbonyl-(C₁-C₆) alkyl radical; an N-Z-amino (C₁-C₆) alkylcarbonyl (C₁-C₆) alkyl radical; an N- (C₁-C₆) alkylamino (C₁-C₆) alkylcarbonyl (C₁-C₆) alkyl radical; an N, N-di (C₁-C₆) alkylamino (C₁-C₆) alkylcarbonyl (C₁-C₆) - alkyl radical; a carboxyl radical; a (C₁-C₆)alkylcarboxyl radical; a C₁-C₆ alkylsulphonyl radical; an aminosulphonyl radical; an N-Z-aminosulphonyl radical; a C₁-C₆ N-alkylaminosulphonyl radical; an N,N-di (C₁-C₆) alkylaminosulphonyl radical; a C₁-C₆ aminosulphonylalkyl radical; a C₁-C₆ N-Z-aminosulphonylalkyl radical; an N- (C₁-C₆) alkylaminosulphonyl (C₁-C₆) alkyl radical; an N, N-di (C₁-C₆) alkylaminosulphonyl (C₁-C₆) alkyl radical; an N-(C₁-C₆)alkylcarbamyl radical; an N,N-di(C₁-C₆)alkylcarbamyl radical; a carbamyl (C₁-C₆) alkyl radical; an N- (C₁-C₆) alkylcarbamyl (C₁-C₆) alkyl radical; an N, N-di (C₁-C₆) alkylcarbamyl (C₁-C₆) alkyl radical; a C₁-C₆ alkyl radical; a C₁-C₆ monohydroxyalkyl radical; a C₂-C₆ polyhydroxyalkyl radical; a (C₁-C₆) alkoxy (C₁-C₆) - alkyl radical; a C₁-C₆ trifluoroalkyl radical; a cyano radical; a group OR₆ or SR₆; or an amino group protected with a (C₁-C₆)alkylcarbonyl, (C₁-C₆) alkylcarboxyl, trifluoro (C₁-C₆) alkylcarbonyl, amino (C₁-C₆) alkylcarbonyl, N-Z-amino (C₁-C₆) alkylcarbonyl, N- (C₁-C₆) alkylamino (C₁-C₆) alkylcarbonyl, N, N-di (C₁-C₆) alkylamino (C₁-C₆) alkylcarbonyl, (C₁-C₆) alkylcarboxyl, N- (C₁-C₆) alkylcarbamyl, N,N-di(C₁-C₆)alkylcarbamyl, C₁-C₆ alkylsulphonyl, aminosulphonyl, N-Z-aminosulphonyl, C₁-C₆ N-alkylaminosulphonyl, N,N-di(C₁-C₆)alkylaminosulphonyl, thiocarbamyl or formyl radical, a group -CO-Z or a group -CO-OZ;
• R₆ denotes a C₁-C₆ alkyl radical; a C₁-C₆ monohydroxyalkyl radical; a C₂-C₆ polyhydroxyalkyl radical; a group Z; a (C₁-C₆) alkoxy (C₁-C₆) alkyl radical; a benzyl radical; a carboxy(C₁-C₆)alkyl radical; a (C₁-C₆)alkylcarboxy(C₁-C₆)alkyl radical; a cyano (C₁-C₆)alkyl radical; a carbamyl (C₁-C₆)alkyl radical; an N-(C₁-C₆) alkylcarbamyl (C₁-C₆) alkyl radical; an N,N-di(C₁-C₆)alkylcarbamyl(C₁-C₆)alkyl radical; a C₁-C₆ trifluoroalkyl radical; a C₁-C₆ aminosulphonylalkyl radical; a C₁-C₆ N-Z-aminosulphonylalkyl radical; an N- (C₁-C₆) alkylaminosulphonyl (C₁-C₆) alkyl radical; an N, N-di (C₁-C₆) alkylaminosulphonyl (C₁-C₆) alkyl radical; a (C₁-C₆) alkylsulphinyl (C₁-C₆) alkyl radical; a (C₁-C₆) alkylsulphonyl (C₁-C₆) alkyl radical; a (C₁-C₆) alkylcarbonyl (C₁-C₆) alkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, formyl, trifluoro (C₁-C₆) alkylcarbonyl, (C₁-C₆) alkylcarboxyl, N- (C₁-C₆) alkylcarbamyl, N,N-di-(C₁-C₆)alkylcarbamyl, thiocarbamyl and C₁-C₆ alkylsulphonyl radicals, and from the groups Z, -CO-Z and -CO-OZ;
• A₁ represents a group -NR₄R₅ or a hydroxyl radical;
• A' represents a group -NR'₄R'₅ or a hydroxyl radical;
• A₂ represents a group -NR₇R₈ or a hydroxyl radical;
• A'₂ represents a group -NR'₇R'₈ or a hydroxyl radical;
• R₄, R₅, R₇, R₈, R'₄, R'₅, R'₇ and R'₈, which may be identical or different, represent a hydrogen atom; a C₁-C₆ alkyl radical; a C₁-C₆ monohydroxyalkyl radical; a C₂-C₆ polyhydroxyalkyl radical; a (C₁-C₆) alkoxy (C₁-C₆) alkyl radical; a benzyl radical; a cyano(C₁-C₆) alkyl radical; a carbamyl (C₁-C₆) alkyl radical; an N- (C₁-C₆) alkylcarbamyl (C₁-C₆) alkyl radical; an N, N-di (C₁-C₆) alkylcarbamyl (C₁-C₆) alkyl radical; a thiocarbamyl(C₁-C₆)alkyl radical; a C₁-C₆ trifluoroalkyl radical; a C₁-C₆ sulphoalkyl radical; a (C₁-C₆)alkylcarboxy(C₁-C₆)alkyl radical; a (C₁-C₆)alkylsulphinyl(C₁-C₆)alkyl radical; a C₁-C₆ aminosulphonylalkyl radical; an N-(C₁-C₆)alkylaminosulphonyl(C₁-C₆)alkyl radical; an N, N-di (C₁-C₆) alkylaminosulphonyl (C₁-C₆) alkyl radical; a (C₁-C₆) alkylcarbonyl (C₁-C₆) alkyl radical; a C₁-C₆ aminoalkyl radical; a C₁-C₆ aminoalkyl radical in which the amine is substituted with one or two identical or different radicals chosen from alkyl, C₁₋C₆ monohydroxyalkyl, C₂-C₆ polyhydroxyalkyl, (C₁-C₆)alkylcarbonyl, N- (C₁-C₆) alkylcarbamyl or N, N-di (C₁-C₆) alkylcarbamyl, C₁-C₆ alkylsulphonyl, formyl, trifluoro (C₁-C₆) alkylcarbonyl, (C₁-C₆) alkylcarboxyl and thiocarbamyl radicals;
• one and only one of the radicals R₄, R₅, R₇, R₈ and/or one and only one of the radicals R'₄, R'₅, R'₇ and R'₈ can also represent a (C₁-C₆)alkylcarboxyl radical; a (C₁-C₆)alkylcarbonyl radical; a formyl radical; a trifluoro(C₁-C₆)alkylcarbonyl radical; an amino(C₁-C₆) alkylcarbonyl radical; an N- (C₁-C₆) alkylamino (C₁-C₆)alkylcarbonyl radical; an N, N-di (C₁-C₆) alkylamino (C₁-C₆) alkylcarbonyl radical; an N- (C₁-C₆)alkylcarbamyl radical; an N,N-di(C₁-C₆)alkylcarbamyl radical; a thiocarbamyl radical; an aminosulphonyl radical; an N-(C₁-C₆)alkylaminosulphonyl radical; an N,N-di (C₁-C₆) alkylaminosulphonyl radical; a (C₁-C₆) alkylsulphonyl radical;
• Z is chosen from the unsaturated cationic groups of formulae (II) and (III) below, and the saturated cationic groups of formula (IV) below: 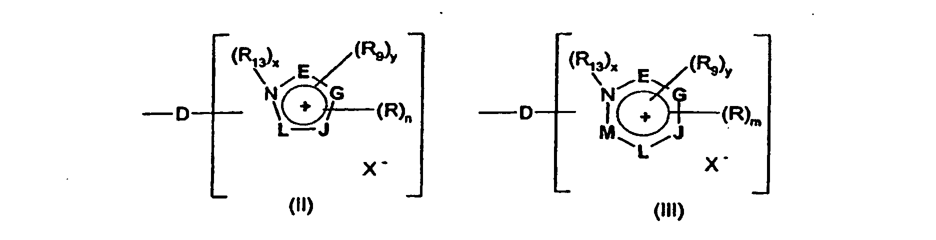 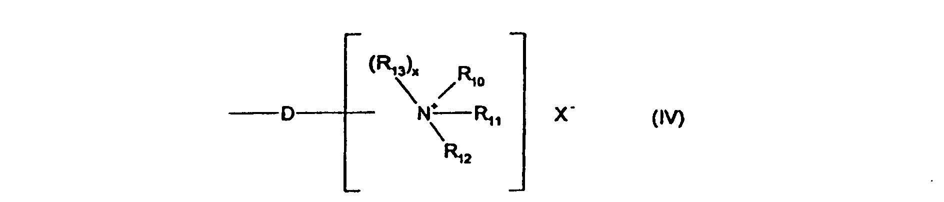
in which:
• D is a linker arm which represents a linear or branched alkylene chain preferably containing from 1 to 14 carbon atoms, which may be interrupted by one or more hetero atoms such as oxygen, sulphur or nitrogen atoms, and which may be substituted with one or more hydroxyl or C₁-C₆ alkoxy radicals;
• the ring members E, G, J, L and M, which are identical or different, represent the atoms required to form a ring system selected from pyrrole, imidazole, pyrazole, oxazole, thiazole, triazole, pyrazolopyrimidinium, pyrazolopyridinium, benzimidazolinium, benzoxazolinium, benzothiazolinium, indolinium, indolidinium, isoindolinium, indazolinium, benzotriazolinium, benzimidazolidinium, and benzopyrimidinium; pyridine, pyrimidine, pyrazine, oxazine, triazine, pyrazolopyrimidinium, quinolinium and tetrahydroquinolinium;
• n is an integer between 0 and 4 inclusive;
• m is an integer between 0 and 5 inclusive;
• the radicals R, which are identical or different, represent a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a C₁-C₆ cyanoalkyl radical, a C₁-C₆ alkoxy radical, a tri (C₁-C₆) alkylsilane (C₁-C₆) alkyl radical, a carboxyl radical, a C₁-C₆ alkylcarbonyl radical, a thio radical, a C₁-C₆ thioalkyl radical, a (C₁-C₆)alkylthio radical, an amino radical, an amino radical protected by a (C₁-C₆)alkyl-carbonyl or (C₁-C₆)alkylsulphonyl radical; a group NHR" or NR"R"' in which R" and R"', which are identical or different, represent a C₁-C₆ alkyl radical, a C₁-C₆ monohydroxyalkyl radical or a C₂-C₆ polyhydroxyalkyl radical;
when n is greater than or equal to 2, two of the adjacent radicals R may also together form a 5- or 6-membered unsaturated ring based on carbon or containing one or more hetero atoms;
• R₉ represents a C₁-C₆ alkyl radical, a C₁-C₆ monohydroxyalkyl radical, a C₂-C₆ polyhydroxyalkyl radical, a C₁-C₆ cyanoalkyl radical, a tri(C₁-C₆)-alkylsilane(C₁-C₆) alkyl radical, a (C₁-C₆)-alkoxy(C₁-C₆)alkyl radical, a C₁-C₆ carbamylalkyl radical, a (C₁-C₆)alkyl-carboxy(C₁-C₆)alkyl radical or a benzyl radical;
• R₁₀, R₁₁ and R₁₂, which may be identical or different, represent a C₁-C₆ alkyl radical; a C₁-C₆ monohydroxyalkyl radical; a C₂-C₆ polyhydroxyalkyl radical; a (C₁-C₆)alkoxy(C₁-C₆) alkyl radical; a cyano(C₁-C₆)alkyl radical; a benzyl radical; a C₁-C₆ amidoalkyl radical; a tri (C₁-C₆) alkylsilane (C₁-C₆)alkyl radical or a C₁-C₆ aminoalkyl radical in which the amine is protected with a (C₁-C₆)alkylcarbonyl or (C₁-C₆)alkylsulphonyl radical; two of the radicals R₁₀, R₁₁ and R₁₂ can also form, together with the nitrogen atom to which they are attached, a 5- or 6-membered saturated carbon-based ring or a ring containing one or more hetero atoms, it being possible for the said ring to be unsubstituted or substituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano (C₁-C₆) alkyl radical, a C₁-C₆ alkoxy radical, a tri (C₁-C₆) alkylsilane (C₁-C₆) alkyl radical, a carboxyl radical, a C₁-C₆ ketoalkyl radical, a thio radical, a C₁-C₆ thioalkyl radical, a (C₁-C₆)alkylthio radical, an amino radical, an amino radical protected with a (C₁-C₆)alkylcarbonyl or (C₁-C₆)alkylsulphonyl radical;
• R₁₃ represents a C₁-C₆ alkyl radical; a C₁-C₆ monohydroxyalkyl radical; a C₂-C₆ polyhydroxyalkyl radical; a benzyl radical; a C₁-C₆ aminoalkyl radical, a C₁-C₆ aminoalkyl radical in which the amine is protected with a (C₁-C₆)alkylcarbonyl, carbamyl or C₁-C₆ alkylsulphonyl radical; a carboxy (C₁-C₆) alkyl radical; a cyano (C₁-C₆) alkyl radical; a carbamyl (C₁-C₆) alkyl radical; a C₁-C₆ trifluoroalkyl radical; a tri (C₁-C₆) alkylsilane (C₁-C₆) alkyl radical; a C₁-C₆ sulphonamidoalkyl radical; a (C₁-C₆) alkylcarboxy (C₁-C₆) alkyl radical; a (C₁-C₆) alkylsulphinyl (C₁-C₆) alkyl radical; a (C₁-C₆) alkylsulphonyl (C₁-C₆) alkyl radical; a (C₁-C₆) alkylketo (C₁-C₆) alkyl radical; an N- (C₁-C₆) alkylcarbamyl (C₁-C₆) alkyl radical; an N- (C₁-C₆) alkylsulphonamido (C₁-C₆) alkyl radical;
• x and y are integers equal to 0 or 1; with the following conditions:
- in the unsaturated cationic groups of formula (II):
- when x = 0, the linker arm D is attached to the nitrogen atom,
- when x = 1, the linker arm D is attached to one of the ring members, E, G, J or L,
- y can adopt the value 1 only:
1) when the ring members E, G, J and L simultaneously represent a carbon atom and when the radical R₉ is carried by the nitrogen atom of the unsaturated ring; or else
2) when at least one of the ring members E, G, J and L represents a nitrogen atom to which the radical R₉ is attached;
- in the unsaturated cationic groups of formula (III):
- when x = 0, the linker arm D is attached to the nitrogen atom,
- when x = 1, the linker arm D is attached to one of the ring members E, G, J, L or M,
- y can adopt the value 1 only when at least one of the ring members E, G, J, L and M represents a divalent atom and when the radical R₉ is carried by the nitrogen atom of the unsaturated ring;
- in the cationic groups of formula (IV):
- when x = 0, then the linker arm is attached to the nitrogen atom bearing the radicals R₁₀ to R₁₂;
- when x = 1, then two of the radicals R₁₀ to R₁₂ form, together with the nitrogen atom to which they are attached, a saturated 5- or 6-membered ring as defined above, and the linker arm B is borne by a carbon atom of the said saturated ring;
• X⁻ represents a monovalent or divalent anion;
it being understood that the number of cationic groups Z is at least equal to 1.

2. Compounds according to the preceding claim, **characterized in that** two of the radicals R₁₀, R₁₁ and R₁₂ form a pyrrolidine ring, a piperidine ring, a piperazine ring or a morpholine ring.

3. Compounds according to either of the preceding claims, **characterized in that** X⁻ is chosen from a halogen atom, a hydroxide, a hydrogen sulphate or a (C₁-C₆)alkyl sulphate.

4. Compounds according to any one of the preceding claims, **characterized in that** they are chosen from:
- 1,4-bis-1-{3-[3-(2,4-diaminophenoxy)propyl]-3N-imidazol-1-ium}butane dichloride monohydrate;
- 1,3-bis[3(2,4-diaminophenoxy)propyl]-3H-imidazol-1-ium chloride;
- 3-[3-(2,4-diaminophenoxy)propyl]-1-[(3-hydroxy-4-methylphenylcarbamoyl)methyl]-3H-imidazol-1-ium chloride;
- 1,4-bis(3-[(3-hydroxy-4-methylphenylcarbamoyl)methyl]-3H-imidazol-1-ium}butane dichloride;
- 1,4-bis[3-(2,4-diaminophenoxy)propyl]-1,4-dimethylpiperazine-1,4-diium dichloride;
- 1,4-bis(3-[3-(2,4-diaminophenyl)ethyl)-3H-imidazol-1-ium}butane dichloride;
- 1-[3-(2,4-diaminophenoxy)propyl]-4-[(3-hydroxy-4-methylphenylcarbamoyl)methyl)-1,4-dimethylpiperazine-1,4-diium dichloride;
- 1,4-bi{3-[2-(3-hydroxy-4-methylphenylamino)ethyl]-3H-imidazol-1-ium}butane dibromide;
- 1,4-bis(3-[(2,4-dihydroxyphenylcarbamoyl)methyl)-3H-imidazol-1-ium}butane dichloride;
- 3-[3-(2,4-diaminophenoxy)propyl]-1-[(2,4-dihydroxyphenylcarbamoyl)methyl]-3H-imidazol-1-ium chloride;
- 4-[2-(2,4-dihydroxyphenyl)-2-oxoethyl)-1-[2-(3-hydroxy-4-methylphenylamino)ethyl]-1,4-dimethylpiperazine-1,4-diium bromide, chloride;
- 1,3-bis([2-(2,4-diaminophenoxy)ethyl]diethylammonium}-propane dibromide; and the addition salts thereof with an acid.

5. Use of the compounds of formula (I) as defined in any one of the preceding claims, as couplers for the oxidation dyeing of keratin fibres, and in particular of human keratin fibres such as the hair.

6. Composition for the oxidation dyeing of keratin fibres and in particular of human keratin fibres such as the hair, **characterized in that** it comprises, in a medium appropriate for dyeing, at least one compound of formula (I) as defined in any one of Claims 1 to 4, as coupler.

7. Composition according to Claim 6, **characterized in that** the compound(s) of formula (I) represent(s) from 0.0005% to 12% by weight relative to the total weight of the dye composition.

8. Composition according to Claim 7, chracterized in that the compound(s) of formula (I) represent(s) from 0.005% to 6% by weight relative to the total weight of the dye composition.

9. Composition according to any one of Claims 6 to 8, **characterized in that** it contains one or more oxidation bases chosen from para-phenylenediamines, bis(phenyl)alkylenediamines, para-aminophenols, ortho-aminophenols and heterocyclic bases.

10. Composition according to Claim 9, **characterized in that** the para-phenylenediamines are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis (β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, and the addition salts thereof with an acid.

11. Composition according to Claim 9, **characterized in that** the bis(phenyl)alkylenediamines are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)-tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis-(ethyl)-N,N'-bis(4'-amino-3'-methylphenyl)ethylenediamine, 1,8-bis(2,5-diaminophenoxy)-3,5-dioxaoctane and the addition salts thereof with an acid.

12. Composition according to Claim 9, **characterized in that** the para-aminophenols are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, 4-amino-2-fluorophenol, and the addition salts thereof with an acid.

13. Composition according to any one of Claims 9 to 12, **characterized in that** the oxidation base(s) represent(s) from 0.0005% to 12% by weight relative to the total weight of the dye composition.

14. Composition according to Claim 13, **characterized in that** the oxidation base(s) represent(s) from 0.005% to 6% by weight relative to the total weight of the dye composition.

15. Composition according to any one of Claims 7 to 14, **characterized in that** it contains an additional coupler chosen from meta-phenylenediamines, meta-aminophenols, meta-diphenols and heterocyclic couplers, and the addition salts thereof with an acid.

16. Composition according to Claim 15, **characterized in that** the additional coupler(s) represent(s) from 0.0001% to 10% by weight relative to the total weight of the dye composition.

17. Composition according to Claim 16, **characterized in that** the additional coupler(s) represent(s) from 0.005% to 5% by weight relative to the total weight of the dye composition.

18. Composition according to any one of Claims 6 to 17, **characterized in that** the addition salts with an acid are chosen from the hydrochlorides, hydrobromides, sulphates, citrates, succinates, tartrates, lactates and acetates.

19. Composition according to any one of Claims 6 to 18, **characterized in that** the medium which is suitable for dyeing (or support) consists of water or a mixture of water and at least one organic solvent chosen from C₁-C₄ lower alkanols, glycerol, glycols and glycol ethers, aromatic alcohols, similar products and mixtures thereof.

20. Composition according to any one of Claims 6 to 19, **characterized in that** it has a pH of between 3 and 12.

21. Process for the oxidation dyeing of keratin fibres, and in particular of human keratin fibres such as the hair, **characterized in that** at least one dye composition as defined in any one of Claims 6 to 20 is applied to these fibres, and **in that** the colour is developed at acidic, neutral or alkaline pH using an oxidizing agent which is added, just at the time of use, to the dye composition, or which is present in an oxidizing composition that is applied separately either simultaneously or sequentially.

22. Process according to Claim 21, **characterized in that** the oxidizing agent is chosen from hydrogen peroxide, urea peroxide, alkali metal bromates, persalts such as perborates and persulphates, and enzymes such as peroxidases and 2-electron oxidoreductases.

23. Multi-compartment dyeing device or multi-compartment dyeing kit, a first compartment of which contains a dye composition as defined in any one of Claims 6 to 20 and a second compartment of which contains an oxidizing composition.
